(19) **Europäisches Patentamt**
**European Patent Office**
**Office européen des brevets**

(11) **EP 0 769 000 B1**

(12) **EUROPEAN PATENT SPECIFICATION**

(45) Date of publication and mention
of the grant of the patent:
**01.12.1999 Bulletin 1999/48**

(21) Application number: **95925194.3**

(22) Date of filing: **15.06.1995**

(51) Int Cl.$^6$: **C07C 53/06**

(86) International application number:
**PCT/SE95/00726**

(87) International publication number:
**WO 96/01249 (18.01.1996 Gazette 1996/04)**

(54) **A PROCESS (II) FOR MAKING POTASSIUM FORMATE**

EIN VERFAHREN ZUR HERSTELLUNG VON KALIUMFORMIAT (II)

UN PROCEDE DE FABRICATION DU FORMIATE DE POTASSIUM (II)

(84) Designated Contracting States:
**AT BE CH DE DK ES FR GB GR IE IT LI LU MC NL PT SE**

(30) Priority: **04.07.1994 SE 9402348**

(43) Date of publication of application:
**23.04.1997 Bulletin 1997/17**

(73) Proprietor: **PERSTORP AB**
**284 80 Perstorp (SE)**

(72) Inventors:
• **ERLANDSSON, Lars**
**S-284 36 Perstorp (SE)**
• **JOHANSSON, Sigvard**
**S-240 36 Stehag (SE)**

(74) Representative:
**Hansen, Bernd, Dr. Dipl.-Chem. et al**
**Hoffmann Eitle,**
**Patent- und Rechtsanwälte,**
**Arabellastrasse 4**
**81925 München (DE)**

(56) References cited:
**EP-A- 0 173 976          DE-A- 4 126 730**
**US-A- 4 277 620**

• **STN INTERNATIONAL, File WPIDS, WPIDS Accession No. 85-084642, NISSAN CHEM. IND. LTD., "Sodium Formate Recovery in Tri Hydric Alcohol Prodn. - by Adding Water-Soluble Solvent to Aq. Phase from Reaction of Sodium Hydroxide with Formaldehyde and/or Para-Formaldehyde"; & JP,A,60 036 432, 25 February 1985.**

## Description

The present invention relates to a process for making potassium formate from formaldehyde and potassium hydroxide in the presence of isobutyric aldehyde.

Potassium formate is, among other applications, used as a component in well servicing fluids, such as drilling fluids, for land based as well as offshore oil drilling. Well drilling depends on many factors, such as the performance of used well servicing fluids. Drilling fluids prevent uncontrolled influx of formation fluids into the well, removes formation cuttings from beneath the bit and transports them to the surface, seals exposed permeable formations, maintains the stability of exposed formations, cools and lubricates the bit and the drill string at points of contact with the cased or uncased drill hole and helps to suspend the weight of the drill string and casing. A number of physical and chemical properties including the rheological properties, of drilling fluids are monitored to ensure satisfactory performance. A major problem is created when the viscosity reduces with increasing temperature. The rheological properties of polymers, included in drilling fluids as for instance viscosifiers, are often substantially degraded or even lost with an increasing temperature. A well known method of stabilising rheological properties is the combination of brines, such as chlorides and formates of alkali metals, and polymers, which increases the critical temperature of the polymer. Potassium formate has been found to exhibit excellent stabilising properties at high temperatures resulting in a critical temperature exceeding 200°C, as disclosed in the research report "High Temperature Stabilisation of Xanthan in Drilling Fluids by the Use of Formate Salts" by J.D. Downs - Koninklijke/Shell Exploratie en Produktie Laboratorium, The Netherlands - Physical Chemistry of Coloids and Interfaces in Production, Paris 1992 pages 197-202. J.D. Downs gives in said paper a detailed disclosure of formates and their stabilising effect. Furthermore, the European Patent Applications 269 939 and 572 113 also disclose the use of formates in drilling fluids.

Potassium formate is very suitable to use as a component in drilling fluids. The salt provides a high density, required for deep well drilling, has a solubility in water exceeding that of other formates and is environmentally safe.

Potassium formate is produced by reacting carbon monoxide with potassium hydroxide in accordance with Reaction I below.

Reaction I:        $CO + KOH \rightarrow HCOOK$

The production method according to Reaction I is disclosed in Enclyclopedia of Chemical Technology, 3rd. edition, volume 18, page 938. The reaction is fairly slow and carried out by absorbing carbon monoxide, freed of acidic gases, in 50-80% by weight of potassium hydroxide at 100-200°C and at a pressure of CO > 690 kPa (> 100 psi). The disadvantages of producing potassium formate in accordance therewith are obvious and can be summarised: High temperature, high pressure and slow reaction. A high yielding, reliable and industrially applicable process for making potassium formate is presently not available and the availability of said compound is thus limited, being for instance a recovered by-product.

Potassium formate can naturally be made by the fundamental but industrially unsuitable or inconvenient reaction between potassium hydroxide and formic acid according to Reaction II below, yielding 1 mole of industrial waste water per mole of potassium formate.

Reaction II:        $HCOOH + KOH \rightarrow HCOOK + H_2O$

The present invention provides a high yielding, reliable and fast process for making potassium formate in an industrially suitable and convenient way. The process yields potassium formate and neopentyl glycol from a reaction between formaldehyde and potassium hydroxide in the presence of isobutyric aldehyde. The reaction is carried out in a liquid, preferably aqueous, state at a molar ratio formaldehyde to potassium hydroxide to isobutyric aldehyde of 1.0:1.0:1.0-3.0:2.0:1.0, such as 1.5:1.2:1.0-2.5:1.7:1.0 or 1.8:1.0:1.0-2.2:1.5:1.0, and at a temperature of 0-100°C, such as 5-90°C or 30-70°C. Yielded potassium formate is suitably recovered by means of for instance a fractional crystallisation, an ion exchanging procedure, an extraction, a phase separation or an electrodialysis.

The reaction according to the process of the invention has a major advantage in being irreversible. The reaction occurs in accordance with Reaction III below, whereby 2 moles of formaldehyde and 1 mole of potassium hydroxide in the presence of 1 mole of isobutyric aldehyde yield 1 mole of potassium formate and 1 mole of neopentyl glycol.

Reaction III:        $2\,HCHO + KOH +$
$$CH_3CH(CH_3)CHO \rightarrow HCOOK +$$
$$CH_2OHC(CH_3)_2CH_2OH$$

The reaction is preferably carried out at atmospheric pressure, but can also advantageously be carried out at a reduced pressure, such as less than 1013 mbar (760 mm Hg) or less than 133.6 mbar (100 mm Hg), or at an increased pressure, such as 1013-2666 mbar (760-2000 mm Hg) or even higher. The reaction temperature is suitably adjusted in relation to the pressure.

The process can preferably be carried out as a batch process, whereby an aqueous potassium hydroxide solution and the isobutyric aldehyde are added to an aqueous formaldehyde solution. The potassium hydroxide solution is, in preferred embodiments, added to the formaldehyde solution during at most 60 minutes, preferably at most 30 minutes, and prior to addition of the isobutyric aldehyde. The isobutyric aldehyde is in such embodiments after the addition of the potassium hydroxide solution added during at most 60 minutes, preferably during 5-30 minutes. The to the addition times subsequent reaction time is normally within the interval of 1-90 minutes, preferably within the interval of 1-30 minutes. The formaldehyde solution has preferably a formaldehyde percentage of 5-65%, such as 10-20% or 30-45%, by weight and corresponding percentage potassium hydroxide is suitably 20-60%, preferably 30-50%, by weight of the potassium hydroxide solution. The process is, when an excess of potassium hydroxide over formaldehyde is used, suitably terminated by a neutralisation with an acid, such as formic acid.

Said addition and reaction time as well as said percentages and neutralisation are of course valid also for other embodiments of the invention not being batch processes, such as a continuous process.

The process according to the invention normally converts at least 90% of the theoretically convertible formaldehyde into potassium formate. The high degree of conversion is a result of the fact that the reaction, as disclosed above, is irreversible.

A further major advantage of the process according to the invention is the yielded neopentyl glycol, which is a commercially valuable product. Neopentyl glycol is an important raw material, direct or indirect, in the production of a large number of substances including saturated and unsaturated polyesters; polyurethane foams, elastomers and resins; synthetic lubricants; thickening agents; antioxidants; and pharmaceuticals. Neopentyl glycol can be recovered from resulting reaction mixture by means of known methods such as distillation, extraction and phase separation. The process gives thus two commercially, chemically and industrially very important compounds.

The major advantages of the process according to the present invention, furthermore, include a fast and simple reaction, a low reaction temperature, atmospheric pressure, a high degree of conversion and no by-products creating waste problems; all enabling a safe, reliable and industrially as well as commercially suitable and convenient production of large quantities of potassium formate.

These and other objects and the attendant advantages will be more fully understood from the detailed description, taken in conjunction with below embodiment example, wherein potassium formate is prepared by an embodiment of the process according to the present invention.

**EXAMPLE**

1014.7 g of an aqueous formaldehyde solution (2.30 moles of formaldehyde) having a formaldehyde content of 6.8% by weight were charged in a reaction vessel equipped with a cooler provided with a receiver, a stirrer, a pressure gauge and means for the heating and cooling of the vessel. 140.3 g of an aqueous potassium hydroxide solution (1.15 mole of potassium hydroxide) having a potassium hydroxide content of 46% by weight were rapidly, ≈ 15 minutes, added to the formaldehyde solution. 73.0 g (1 mole) of a 98.6% isobutyric aldehyde were subsequent the addition of potassium hydroxide during 37 min. added to obtained formaldehyde/potassium hydroxide mixture.

The temperature increased exotherically during the addition of isobutyric aldehyde from ≈ 23°C to 46°C and the vessel were cooled to maintain a temperature of 46°C.

The reaction was allowed to continue at 46°C for 5 minutes. The vessel walls were now rinsed with a minor amount of water and the reaction mixture neutralised by addition of 2 g of formic acid with a concentration of 85%.

The process resulted in 91.5 g of potassium formate and 102.3 g of neopentyl glycol.

The recovered quantity of potassium formate can be derived as follows:

3.6 g - yielded from neutralisation with formic acid. 87.9 g - yielded from the reaction between formaldehyde and potassium hydroxide. Equals a conversion of ≈ 90.9% of the formaldehyde to potassium formate (possible losses in connection with reaction, material handling and analyses are not regarded). The theoretical amount is 96.7 g = 1.15 mole.

**Claims**

1. A process for making potassium formate **characterised in**, that
   the potassium formate and neopentyl glycol yield from a reaction between formaldehyde and potassium hydroxide in the presence of isobutyric aldehyde, the reaction being carried out in a liquid, preferably aqueous, state at a molar ratio formaldehyde to potassium hydroxide to isobutyric aldehyde of 1.0:1.0:1.0 to 3.0:2.0:1.0, such as 1.5:1.2:1.0 to 2.5:1.7:1.0 or 1.8:1.0:1.0 to 2.2:1.5:1.0, and at a temperature of 0-100°C, preferably 30-70°C.

2. A process according to claim 1 **characterised in**, that
   yielded potassium formate is recovered by means of an ion exchanging procedure, an extraction, a phase separation, an electrodialysis or a fractional crystallisation.

3. A process according to claim 1 or 2 **characterised in**, that

the reaction is carried out at atmospheric pressure.

4. A process according to claim 1 or 2
   **characterised in**, that
   the reaction is carried out at a pressure of less than
   1013 mbar (760 mm Hg), preferably less than 133.6
   mbar (100 mm Hg).

5. A process according to claim 1 or 2
   **characterised in**, that
   the reaction is carried out at a pressure of more than
   1013 mbar (760 mm Hg) and preferably less than
   2666 mbar (2000 mm Hg).

6. A process according to any of the claims 1 - 5
   **characterised in**, that
   the process is a batch process, whereby an aqueous potassium hydroxide solution and isobutyric aldehyde are added to an aqueous formaldehyde solution.

7. A process according to claim 6
   **characterised in**, that
   the potassium hydroxide solution prior to addition of the isobutyric aldehyde is added to the formaldehyde solution during at most 60, preferably during at most 30, minutes.

8. A process according to claim 7
   **characterised in**, that
   isobutyric aldehyde subsequent addition of the potassium hydroxide solution is added during at most 60, preferably during 5-30, minutes.

9. A process according to any of the claims 6 - 8
   **characterised in**, that
   the formaldehyde solution has a formaldehyde percentage of 5-65%, such as 10-20% or 30-45%, by weight.

10. A process according to any of the claims 6 - 9
    **characterised in**, that
    the potassium hydroxide solution has a potassium hydroxide percentage of 20-60%, preferably 30-50%, by weight.

11. A process according to any of the claims 6 - 10
    **characterised in**, that
    the process is carried out at a molar ratio formaldehyde to potassium hydroxide of 1:1 to 2:1 and that the process is terminated by neutralisation with an acid, preferably formic acid.

12. A process according to any of the claims 1 - 11
    **characterised in**, that
    at least 90% of theoretically convertible formaldehyde is converted into potassium formate.

**Patentansprüche**

1. Verfahren zur Herstellung von Kaliumformiat dadurch **gekennzeichnet**, daß
   die Reaktion zwischen Formaldehyd und Kaliumhydroxid in der Gegenwart von Isobutyraldehyd Kaliumformiat und Neopentylglykol ergibt, wobei die Reaktion im flüssigen, vorzugsweise wäßrigen, Zustand bei einem Molverhältnis Formaldehyd:Kaliumhydroxid:Isobutyraldehyd von 1,0:1,0:1,0 bis 3,0:2,0:1,0, beispielsweise bei 1,5:1,2:1,0 bis 2,5:1,7:1,0 oder 1,8:1,0:1,0 bis 2,2:1,5:1,0, und bei einer Temperatur von 0 bis 100°C, vorzugsweise 30 bis 70°C ausgeführt wird.

2. Verfahren gemäß Anspruch 1,
   dadurch **gekennzeichnet**, daß
   das resultierende Kaliumformiat mittels eines Ionenaustauschverfahrens, einer Extraktion, einer Phasentrennung, einer Elektrodialyse oder einer fraktionierten Kristallisation rückgewonnen wird.

3. Verfahren gemäß Anspruch 1 oder 2,
   dadurch **gekennzeichnet**, daß
   die Reaktion bei atmosphärischem Druck ausgeführt wird.

4. Verfahren gemäß Anspruch 1 oder 2,
   dadurch **gekennzeichnet**, daß
   die Reaktion bei einem Druck von weniger als 1013 mbar (760 mmHg), vorzugsweise bei weniger als 133,6 mbar (100 mmHg) ausgeführt wird.

5. Verfahren gemäß Anspruch 1 oder 2,
   dadurch **gekennzeichnet**, daß
   die Reaktion bei einem Druck von mehr als 1013 mbar (760 mmHg) und vorzugsweise bei weniger als 2666 mbar (2000 mmHg) ausgeführt wird.

6. Verfahren gemäß einem der Ansprüche 1 bis 5,
   dadurch **gekennzeichnet**, daß
   das Verfahren ein Batch-Verfahren ist, wobei man eine wäßrige Kaliumhydroxid-Lösung und Isobutyraldehyd zu einer wäßrigen Formaldehyd-Lösung gibt.

7. Verfahren gemäß Anspruch 6,
   dadurch **gekennzeichnet**, daß
   die Kaliumhydroxid-Lösung vor der Zugabe des Isobutyraldehyds zu der Formaldehyd-Lösung während maximal 60, vorzugsweise maximal 30 min gegeben wird.

8. Verfahren gemäß Anspruch 7,
   dadurch **gekennzeichnet**, daß
   der Isobutyraldehyd nachfolgend zur Zugabe der Kaliumhydroxid-Lösung während maximal 60, vorzugsweise während 5 bis 30 min zugegeben wird.

9. Verfahren gemäß einem der Ansprüche 6 bis 8, dadurch **gekennzeichnet**, daß die Formaldehyd-Lösung einen Formaldehyd-Prozentsatz von 5 bis 65 %, beispielsweise 10 bis 20 % oder 30 bis 45 %, per Gewicht aufweist.

10. Verfahren gemäß einem der Ansprüche 6 bis 9, dadurch **gekennzeichnet**, daß die Kaliumhydroxid-Lösung einen Kaliumhydroxid-Prozentsatz von 20 bis 60 %, vorzugsweise 30 bis 50 %, per Gewicht aufweist.

11. Verfahren gemäß einem der Ansprüche 6 bis 10, dadurch **gekennzeichnet**, daß das Verfahren bei einem Molverhältnis Formaldehyd zu Kaliumhydroxid von 1:1 bis 2:1 durchgeführt wird und daß das Verfahren durch Neutralisieren mit einer Säure, vorzugsweise Ameisensäure, beendet wird.

12. Verfahren gemäß einem der Ansprüche 1 bis 11, dadurch **gekennzeichnet**, daß mindestens 90 % des theoretisch umwandelbaren Formaldehyds zu Kaliumformiat umgewandelt werden.

**Revendications**

1. Procédé pour fabriquer du formiate de potassium, caractérisé en ce que le formiate de potassium et du néopentyle glycol sont obtenus par réaction entre du formaldéhyde et de l'hydroxyde de potassium en présence d'aldéhyde isobutyrique, la réaction étant effectuée dans un état liquide, de préférence aqueux, à un rapport en moles formaldéhyde sur hydroxyde de potassium sur aldéhyde isobutyrique de 1,0 : 1,0 : 1,0 à 3,0 : 2,0 : 1,0, comme par exemple de 1,5 : 1,2 : 1,0 à 2,5 : 1,7 : 1,0 ou de 1,8 : 1,0 : 1,0 à 2,2 : 1,5 : 1,0 et à une température de 0 - 100 °C et, de préférence, de 30 - 70 °C.

2. Procédé selon la revendication 1, caractérisé et ce que le formiate de potassium produit est récupéré par un procédé d'échange d'ions, une extraction, une séparation de phases, une électrodialyse ou une cristallisation fractionnée.

3. Procédé selon la revendication 1 ou la revendication 2, caractérisé en ce que la réaction est effectuée à pression atmosphérique.

4. Procédé selon la revendication 1 ou 2, caractérisé en ce que la réaction est effectuée à une pression inférieure à 1013 mbar (760 mm Hg) et, de préférence, inférieure à 133,6 mbar (100 mm Hg).

5. Procédé selon la revendication 1 ou 2, caractérisé en ce que la réaction est effectuée à une pression supérieure à 1013 mbar (760 mm Hg) et, de préférence, inférieure à 2666 mbar (2000 mm Hg).

6. Procédé selon l'une quelconque des revendications 1 - 5, caractérisé en ce que le procédé est un procédé discontinu, dans lequel une solution aqueuse d'hydroxyde de potassium et de l'aldéhyde isobutyrique sont ajoutés à une solution aqueuse de formaldéhyde.

7. Procédé selon la revendication 6, caractérisé en ce que la solution d'hydroxyde de potassium avant l'addition de l'aldéhyde isobutyrique est ajoutée à la solution de formaldéhyde sur une durée maximale de 60 minutes et, de préférence, sur une durée maximale de 30 minutes.

8. Procédé selon la revendication 7, caractérisé en ce que l'aldéhyde isobutyrique, après l'addition de la solution d'hydroxyde de potassium, est ajouté sur une durée maximale de 60 minutes et, de préférence, sur une durée de 5 - 30 minutes.

9. Procédé selon l'une quelconque des revendications 6 - 8, caractérisé en ce que la solution de formaldéhyde a un pourcentage de formaldéhyde de 5 - 65 %, par exemple de 10 - 20 % ou de 30 - 45 % en poids.

10. Procédé selon l'une quelconque des revendications 6 - 9, caractérisé en ce que la solution d'hydroxyde de potassium a un pourcentage d'hydroxyde de potassium de 20 - 60 % et, de préférence de 30 - 50 % en poids.

11. Procédé selon l'une quelconque des revendications 6 - 10, caractérisé en ce que le procédé est effectué avec un rapport en moles formaldéhyde sur hydroxyde de potassium de 1 : 1 à 2 :1 et en ce que la procédé est arrêté par neutralisation avec un acide, de préférence l'acide formique.

12. Procédé selon l'une quelconque des revendications 1 - 11, caractérisé en ce qu'au moins 90 % du formaldéhyde théoriquement convertible sont convertis en formiate de potassium.